# EUROPEAN PATENT SPECIFICATION

(11) **EP 2 094 178 B1**
(45) Date of publication and mention of the grant of the patent: **23.01.2013**
(21) Application number: 07865531.3
(22) Date of filing: 12.12.2007
(51) Int. Cl.: A61B 17/82, A61B 17/88, A61B 17/00

(54) **WIRE TWISTER**
DRILLAPPARAT
CERCLEUR

(30) Priority: 12.12.2006 US 874264 P
(43) Date of publication of application: 02.09.2009
(73) Proprietor: Synthes GmbH, 4436 Oberdorf (CH)
(72) Inventor: DELL'OCA, Alberto A. Fernandez, Montevideo, 11500 (UY)
(74) Representative: Lusuardi, Werther
(86) International application number: PCT/US2007/087129
(87) International publication number: WO 2008/073947

(56) References cited:
- EP-A- 0 625 336
- EP-A- 1 192 908
- GB-A- 2 214 113
- US-A- 2 943 650
- US-B1- 6 752 810

## Description

The present invention relates to a device for twisting a wire around bone according to the preamble of claim 1.

The use of wire to stabilize fractures and twisting the wire to secure it to the reduced fracture is a well-known technique.

Known tools to twist the wire do not permit tension to be released once it has been applied. Furthermore, these known tools increase tension as the wire is twisted which may cause the wire to fail.

A wire passer as disclosed in U.S. Patent Appln. No. 11/194,642, to A. Fernández Dell' Oca and entitled "Two Member Cerclage Tool" permits the looping of wire about a bone via a minimal incision. The known wire twisting devices require substantial incisions and, therefore, involve significant muscle and tissue trauma. From US Patent No. 2,943,650 a device for forming and closing a tight loop of wire is known, which is based on the preamble of claim 1.

The present invention provides a wire twister permitting release of a previously applied tension should it be needed to improve bone reduction.

The present invention further provides a wire twister including a mechanism which, while twisting the wire, maintains a substantially constant tension on the wire and which is useable via a small incision on the order of size required for minimally invasive ostheosyntesis.

A wire twister according to one embodiment of the invention comprises a long tube which may be inserted to the bone via a minimal incision. A wire is passed to the bone through the tube which includes a main peg which twists the wire as the wire is rotated. The wire twister also includes a wire holder to which both ends of the wire are attached. The wire holder is located in a part of the tube which remains outside the incision during the procedure. A wire pulling mechanism pulls the wire proximally thereby tensioning it, but also allows this tension to be released to obtain appropriate reduction of the fracture. The wire pulling mechanism according to one embodiment includes a flexible ratchet while these mechanisms maintain substantially constant tension on the wire while it is being twisted.

The invention and developments of the invention are described in greater detail below using partially schematic illustrations.

### BRIEF DESCRIPTION OF THE VIEWS OF THE DRAWINGS

Fig. 1 illustrates a perspective view of prior art; and
Fig. 2 shows a longitudinal cross section of the first embodiment of the present invention.

### DETAILED DESCRIPTION

The present invention, which may be further understood with reference to the following description and the appended drawings, relates to devices for treating fractures and, in particular, relates to internal fixation devices for treating fractures along a length of a bone. Exemplary embodiments of the present invention provide a tool for twisting a wire or cable after it has been looped around a fractured bone while compensating for the increase in tension associated with the twisting of the cable to maintain a substantially constant tension on the cable or wire. Those skilled in the art will understand that, although this application describes looping wire and cable about a bone and twisting this cable or wire, any suitably strong and biocompatible filament or group of filaments may be substituted for the described wire or cable without deviating from the invention.

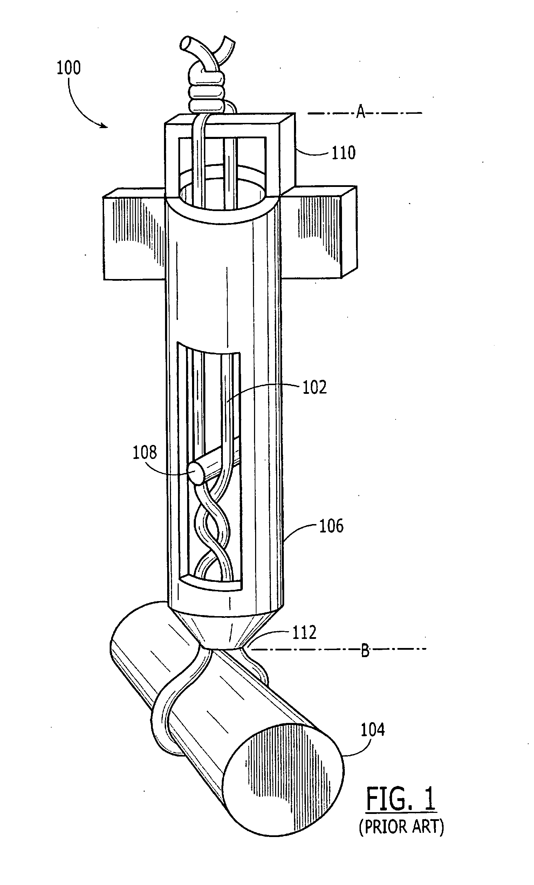

Fig. 1 shows a perspective view of a prior art device 100 for twisting a wire 102 to reduce a fractured bone 104. The device 100 comprises a tube 106, a main peg 108 that guides the twisting of the wire 102, and a wire holder 110 to which both ends of the wire 102 are attached. As shown in Fig. 1, a distance from the wire holder 110 to a distal end 112 of the tube 106 (A to B) is constant and is unable to compensate for increasing tension as the wire 102 is twisted. Thus, once a wire 102 has been passed around a fractured bone 104 and a desired tension has been obtained, further twisting of the wire 102 will increase the tension beyond the desired tension, preventing proper fracture reduction.

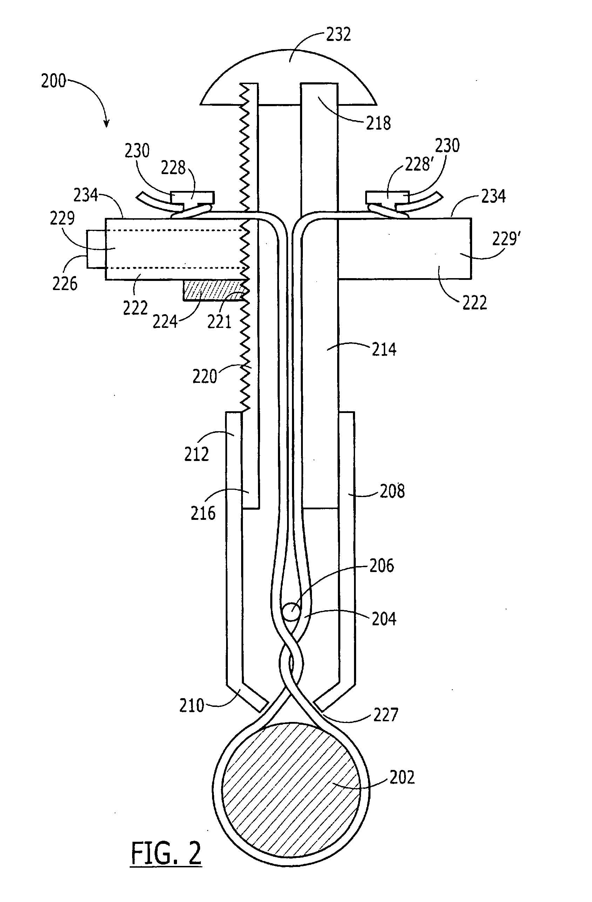

As shown in Fig. 2 a device 200 according to a first exemplary embodiment of the present invention comprises a main tube 208 and an inner tube 214 including a geared surface 220. The inner tube 214 also includes a pulling handle 222 coupled to a lever 224 engaging teeth of the geared surface 220. A loop of wire 204 extends from a first end coupled to a first wire holder 228 on a first arm 229 of the pulling handle 222 through the main tube 208 passing by a peg 206 and exiting an opening 227 at a distal end 210 of the device 200. The wire 204 loops around the bone 202, reenters the tube 208 and extends past the peg 26 on the opposite side from the other end of the wire 204 to a second wire holder 228' on the other arm 229' of the pulling handle 222.

The main tube 208 extends from the distal opening 227 to a proximal opening 212 which receives the distal end of the inner tube 214. The ends of the wire 204 pass through a distal opening 216 into the inner tube 214 and extend proximally through the inner tube 214 to side openings in the inner tube 214 through which they pass to the wire holders 228, 228'. The distal opening 227 is preferably narrower in diameter than the proximal opening 212 with a the diameter of the distal opening 227 being selected so that portions of the wire 204 passing therethrough are brought closer to one another. The distal opening 216 is received within and fixed to the proximal opening 212 of main tube 208 such that the wire 7 may pass through both the main tube 208 and a portion of the inner tube 214. A proximal opening 218 of the inner tube 214 in this embodiment is closed by an element 232 so that, when pulling the handle 222 along a length of the inner tube 214 to tension the wire 204, the pulling handle 222 is not inadvertently pulled off of the inner tube 214.

The geared surface 220 formed on a portion of an outer surface of the inner tube 214 engages a corresponding geared surface 221 on a radially inner surface of a lever 224 coupled to the ann 229 to lock the arms 229, 229' of the pulling handle 222 in a desired position relative to the tube 214. The arm 229 includes a button 226 extending therethrough to engage the geared surface 220 so that, pushing the button 226 toward the axis of the tube 214 bends the geared surface 220 out of engagement with the geared surface 221 so that the arms 229, 229' may be moved proximally and distally along the tube 214. As would be understood by those skilled in the art, the geared surface 220 is preferably sufficiently flexible that it may be moved radially inward out of engagement with the lever 224 through the action of the button 226 and so that, when the wire 204 is twisted, increasing tension in the wire 204 and pulling the pulling handle 222 distally, the geared surface 220 may bend to release the lever 224 from the geared surface 220 allowing the pulling handles 222 to slide along the inner tube 214. The flexibility of the geared surface 220 is preferably selected based on a degree of tension desired for the device to be able to withstand. For example, as would be understood by those of skill in the art, a thickness of a wall of the tube 214 forming the geared surface 220 may be made thicker or thinner relative to other portions of the tube 214 to achieve the desired bending characteristics. The geared surface 220 preferably extends along portion of a length of the inner tube 214 corresponding to a desired range of motion of the arms 229, 229' relative to the tube 214. Furthermore, as would be understood, the geared surface 220 preferably extends around only a portion of the circumference of the inner tube 214. However, in an alternative embodiment, the geared surface 220 may extend around the entire circumference of the tube 214 so long as the arm 229' is either maintained unlocked with respect to the geared surface 220 or is unlockable (e.g., via a mechanism similar to that on the arm 229). The teeth of the geared surface 220 may be angled distally such that the pulling handle 222 may be more easily pulled proximally toward the end 218 of the inner tube 214, but will remain locked in place as the pulling handle 222 is pulled distally (e.g., by the tension in the wire 204) toward the distal end 216 of the inner tube 214.

Each of the arms 229, 229' of the pulling handle 222 includes a corresponding wire holder 228, 228', respectively, for securing the opposite ends of the wire 204 thereto after passing through the main tube 208 and a portion of the inner tube 214. Each of the wire holders 228, 228' may, for example, include a vertical element with a shoulder 230 at its proximal end preventing the wire 204 from slipping off after being secured thereto. The wire holders 228, 228' may be positioned on proximal surfaces 234 of the arms 229, 229', respectively, such that the pulling handle 222 may withstand some of the tension of the wire 204 as the pulling handle 222 is pulled proximally to increase tension in the wire 204. The wire 204 may be secured to the wire holders 228, 228' by bending, twisting or tying the ends of the wire 204 around the wire holders 228, 228' distally of the shoulder 230 to prevent slipping. As each end of the wire 204 is secured to a separate wire holder 228, 228' movement of the handles 222 relative to the tube 214 generates equal tension in both ends of the wire 204.

The peg 206 which extends within the main tube 208 between the ends of the wire 204 guides the twisting of the wire 204. As the device 200 is rotated relative to the bone 202, the ends of the wire 204 are twisted about one another distally of the peg 206, as shown in Fig. 1. This twisting tends to increase the tension in the wire 204. However, this increased tension bows the geared surface 220 bringing it out of engagement with the geared surface 221, allowing the handle 222 to move proximally relative to the tube 214 until equalization of the tension allows the geared surface 220 to straighten itself and re-engage the geared surface 221 locking the handle 222 in a position which maintains the tension in the wire 204 substantially constant at the desired tension. That is, the stiffness of the tube 214 and, in particular, the geared surface 220, is selected so that tensions above a desired level will bend the geared surface 220 out of contact with the geared surface 221.

In an exemplary surgical technique which does not form part of the invention, a wire 204 is looped around a fractured bone 202 via a small skin incision. After the wire 204 has been looped around the bone 202, the ends of the wire 204 are passed through the main tube 208 by inserting the wire 204 at the distal opening 227 and passing each end of the wire 204 on opposite sides of the peg 206 through at least a portion of the inner tube 214 via the distal opening 216. A diameter of the main tube 208 may preferably range from 6mm to 10mm, while a length of the main tube may preferably range from 150mm to 200 mm. The ends of the wire 204 are then secured to the wire holders 228, 228' on opposite sides of the pulling handle 222. The pulling handle 222 is then pulled proximally relative to the tube 214 (i.e., toward the proximal end 218 of the tube 214) such that the ends of the wire 204 are pulled away from the bone 202 until a desired tension in the wire 204 is obtained. The desired tension is maintained through the wire 204 by the lever 224 which engages the upwardly angled teeth of the geared surface 220. If it is determined that some tension needs to be released, the button 226 is pressed to disengage the geared surfaces 220, 221 from one another allowing the pulling handle 222 to be moved distally to release tension from the wire 204. The pulling handle 222 may be moved proximally and distally as necessary (up to the maximum tension permitted before the geared surface 220 is bent out of engagement with the geared surface 221) to achieve the desired tension within the wire 204 and reduce the fracture of the bone 202.

After the desired tension has been obtained, the wire 204 is twisted to fix the reduced fracture. As described above, the wire 204 may be twisted by rotating the pulling handles 222 and the tubes 208 and 214 about a longitudinal axis of the tube 214. Since the ends of the wire 204 are secured to the arms 229, 229' of the wire holder 228, the ends of the wire 204 are turned as the pulling handle 222 is rotated. The turning of the ends of the wire 204 twists the wire 204 distally of the peg 206. The peg 206 guides the twisting of the wire 204 by maintaining the twists of wire 204 distal to the peg 206 such that the wire 204 located proximally of the peg 206 does not become entangled and wire 204 is not loosely twisted along its entire length. If the tension level is increased beyond a desired level (e.g., during twisting), the tension may be moved gradually back to the desired level by pressing the button 226 to disengage the geared surface 220 from the geared surface 221 and moving the handle 222 distally until the desired tension is achieved once again. As would be understood by those skilled in the art, such adjustments may be made periodically as the wire 204 is twisted to reduce the possibility of failure of the wire 204 which may occur if the maximum tension sustainable by the wire 204 is exceeded. As described above, if the desired tension is substantially equal to the maximum tension which may be applied by the device 200, this adjustment will be made automatically as the geared surface 220 bends out of engagement allowing the handle 222 to slip distally to reestablish the desired pressure.

## Claims

1. A device (200) for twisting wire looped about a bone (202), the device comprising:
a longitudinal member (208,214) sized for insertion through an incision to a target location adjacent a bone (202), the longitudinal member (208,214) defining a passage extending therethrough to a distal opening (227) sized to receive beside one another end portions of a wire (204) to be twisted about a bone (202);
a cross-member (206) extending across the passage, a clearance within the passage on either side of the cross-member being sized to receive one end portion of a wire (204) to be twisted about the bone; and
first and second wire mounting units (228;228') on substantially opposite sides of a longitudinal axis of the passage for coupling to first and second ends, respectively, of a wire (204) be twisted about a bone (202), the first and second wire mounting units (228;228') being proximal of the cross-member (206) and coupled to the longitudinal member (208,214) for movement relative thereto along the axis,
**characterized in that**
the device (200) further comprises a handle (222) extending laterally from the longitudinal member (208,214) with the wire mounting units (228;228') on opposite arms (229,229') thereof.

2. The device of claim 1, further including a first geared surface on the handle that releaseably engages a second geared surface on the longitudinal member.

3. The device of claim 2, wherein a stiffness of a portion of the longitudinal member including the second geared surface is selected so that, when the wire applies a predetermined tension to the wire mounting units, the second geared surface bends out of engagement with the first geared surface.

4. The device of claim 2, further including a release mechanism on the handle that is manually operable to move the first and second geared surfaces out of engagement with one another.

5. The device of claim 1, wherein the longitudinal member is formed of first and second substantially tubular members, the first tubular member extending from the distal opening to an open proximal end in which a distal end of the second tubular member is received.

6. The device of claim 5, further including a first geared surface on the handle that releasably engages a second geared surface on the second tubular member.

7. The device of claim 1 wherein a diameter of the longitudinal member ranges from 6 mm to 10 mm.

8. The device of claim 1, wherein a length of the longitudinal member ranges from 150 mm to 200 mm.

## Patentansprüche

1. Vorrichtung (200) zum Verdrillen eines einen Knochen (202) umschlingenden Drahts, wobei die Vorrichtung umfasst:
ein Längselement (208, 214), welches zum Einführen durch eine Inzision an einen an einen Knochen (202) angrenzenden Zielort bemessen ist, wobei das Längselement (208, 214) einen Durchgang definiert, welcher sich durch dasselbe hindurch zu einer distalen Öffnung (227) erstreckt, welche zur Aufnahme von Endabschnitten eines um einem Knochen zu windenden Drahtes (204) nebeneinander bemessen ist;
ein Querelement (206), welches sich über den Durchgang erstreckt, wobei ein Freiraum innerhalb des Durchgangs auf beiden Seiten des Querelements so bemessen ist, dass ein Endabschnitt eines um einem Knochen zu windenden Drahts (204) darin aufgenommen wird; und
erste und zweite Drahtbefestigungseinheiten (228, 228') auf im Wesentlichen gegenüberliegenden Seiten einer Längsachse des Durchgangs zur Verbindung mit ersten und zweiten Enden eines um einem Knochen (202) zu windenden Drahtes (204), wobei die erste und zweite Drahtbefestigungseinheiten (228, 228') proximal zum Querelement (206) angeordnet sind und mit dem Längselement (208, 214) zur Bewegung relativ dazu entlang der Achse gekoppelt sind,
**dadurch gekennzeichnet, dass**
die Vorrichtung (200) zusätzlich einen Griff (222) umfasst, der sich seitlich vom Längselement (208, 214) mit den Drahtbefestigungseinheiten (228, 228') auf gegenüberliegenden Armen (229, 229') desselben ausdehnt.

2. Vorrichtung nach Anspruch 1, **dadurch gekennzeichnet, dass** die Vorrichtung zusätzlich am Handgriff eine erste verzahnte Oberfläche umfasst, die lösbar in eine zweite verzahnte Oberfläche am Längselement eingreift.

3. Vorrichtung nach Anspruch 2, **dadurch gekennzeichnet, dass** eine Steifigkeit eines die zweite verzahnte Oberfläche einschliessenden Teils des Längselements so gewählt ist, dass, wenn der Draht eine vorbestimmte Spannung auf die Drahtbefestigungseinheiten ausübt, die zweite verzahnte Oberfläche aus dem Eingriff mit der ersten verzahnten Oberfläche ausbiegt.

4. Vorrichtung nach Anspruch 2, **dadurch gekennzeichnet, dass** die Vorrichtung zusätzlich am Griff eine Auslösevorrichtung umfasst, die manuell betätigbar ist, um die erste und zweite verzahnte Oberfläche aus dem Eingriff miteinander zu bewegen.

5. Vorrichtung nach Anspruch 1, **dadurch gekennzeichnet, dass** das Längselement aus ersten und zweiten im Wesentlichen rohrförmigen Elementen gebildet ist, wobei das erste röhrenförmige Element sich von der distalen Öffnung zu einem offenen proximalen Ende ausdehnt, in welchem ein distales Ende des zweiten rohrförmigen Elements aufgenommen ist.

6. Vorrichtung nach Anspruch 5, **dadurch gekennzeichnet, dass** die Vorrichtung zusätzlich eine erste verzahnte Oberfläche am Griff umfasst, die lösbar in eine zweite verzahnte Oberfläche am zweiten rohrförmigen Element eingreift.

7. Vorrichtung nach Anspruch 1, **dadurch gekennzeichnet, dass** ein Durchmesser des Längselements im Bereich von 6 mm bis 10 mm liegt.

8. Vorrichtung nach Anspruch 1, **dadurch gekennzeichnet, dass** eine Länge des Längselements im Bereich von 150 mm bis 200 mm liegt.

## Revendications

1. Dispositif (200) pour torsader un fil métallique formant boucle autour d'un os (202), le dispositif comprenant :
un élément longitudinal (208, 214) dimensionné en vue d'une insertion à travers une incision vers une position cible adjacente à un os (202), l'élément longitudinal (208, 214) définissant un passage s'étendant à travers lui jusqu'à une ouverture distale (227) dimensionnée pour recevoir, à côté l'une de l'autre, des parties d'extrémité d'un fil métallique (204) à torsader autour d'un os (202) ;
un élément transversal (206) s'étendant à travers le passage, un espace libre situé à l'intérieur du passage sur l'un et l'autre côté de l'élément transversal étant dimensionné pour recevoir une partie d'extrémité d'un fil métallique (204) à torsader autour de l'os ; et
un premier et un second systèmes de fixation de fil métallique (228 ; 228') placés essentiellement sur les côtés opposés d'un axe longitudinal du passage en vue d'être couplés à la première et à la seconde extrémités, respectivement, d'un fil métallique (204) à torsader autour d'un os (202), le premier et le second systèmes de fixation de fil métallique (228 ; 228') étant proximaux de l'élément transversal (206) et couplés à l'élément longitudinal (208, 214) en vue d'un déplacement par rapport à celui-ci le long de l'axe, **caractérisé en ce que**
le dispositif (200) comprend, de plus, une poignée (222) s'étendant latéralement à partir de l'élément longitudinal (208, 214) avec les systèmes de fixation du fil métallique (228 ; 228') monté sur ses bras opposés (229 ; 229').

2. Dispositif selon la revendication 1, comprenant, de plus, une première surface dentée formée sur la poignée qui s'engage de façon libérable avec une seconde surface dentée formée sur l'élément longitudinal.

3. Dispositif selon la revendication 2 dans lequel la rigidité d'une partie de l'élément longitudinal comportant la seconde surface dentée est sélectionnée de telle sorte que, lorsque le fil métallique applique une tension prédéterminée aux systèmes de fixation de fil métallique, la seconde surface dentée se libère en se courbant de l'engagement avec la première surface dentée.

4. Dispositif selon la revendication 2 comprenant, de plus, un mécanisme de relâchement placé sur la poignée qui peut être actionné à la main afin de déplacer les première et seconde surfaces dentées hors de leur engagement l'une avec l'autre.

5. Dispositif selon la revendication 1, dans lequel l'élément longitudinal est formé d'un premier et d'un second éléments tubulaires, le premier élément tubulaire s'étendant à partir de l'ouverture distale jusqu'à une extrémité proximale ouverte dans laquelle une extrémité distale du second élément tubulaire est reçue.

6. Dispositif selon la revendication 5 comprenant, de plus, une première surface dentée formée sur la poignée qui s'engage de façon libérable avec une seconde surface dentée formée sur le second élément tubulaire.

7. Dispositif selon la revendication 1 dans lequel le diamètre de l'élément longitudinal se situe dans une plage allant de 6 mm à 10 mm.

8. Dispositif selon la revendication 1, dans lequel la longueur de l'élément longitudinal se situe dans une plage allant de 150 mm à 200 mm.
